# EUROPEAN PATENT APPLICATION

(11) **EP 4 566 542 A1**
(43) Date of publication of application: **11.06.2025**
(21) Application number: 23215225.6
(22) Date of filing: 08.12.2023
(51) Int. Cl.: A61B 6/03, A61B 5/055, A61B 6/46, G01R 33/48

(54) **MEDICAL SCANNER**

(71) Applicant: Koninklijke Philips N.V., 5656 AG Eindhoven (NL)
(72) Inventor: MOTIYAR, Rahul, Eindhoven (NL); KNOESTER, Jaap, Eindhoven (NL); NOTENBOOM, Masja, 5656AG Eindhoven (NL)
(74) Representative: Philips Intellectual Property & Standards

(57) **Abstract**

A medical scanner comprises a scanning system with a scanner housing having a bore through which a patient is moved during a scan. An instruction system is used for providing breathing instructions to the patient during a scan. The instruction system comprises a colored lighting strip which extends from outside the bore at opposite sides of the housing through the bore. In this way, intuitive instructions can be provided as color outputs, and they can be seen by the patient while the patient is moved through the bore.

## Description

### FIELD OF THE INVENTION

This invention relates to medical scanners, and in particular it relates to medical scanning which requires a user to perform a breath hold.

### BACKGROUND OF THE INVENTION

There are several types of exams which are supposed to be performed during a breath hold, such as chest and abdomen CT scans, and scans for cardiac and pulmonary embolism cases. The breath hold is used to avoid corruption of image quality by motion artefacts.

Known CT scanners are equipped with three indicators, for "Breathe in", Breath hold" and Breathe out". The patient is instructed by the operator or by auto-voice commands to take a deep breath and the breath indicator is illuminated. The next instruction is to hold breath for the duration of the scan and the breath hold indicator is illuminated. Once the scan is complete, the breathe out indicator is illuminated, along with a voice instruction to alert the patient that the breath hold period is ended.

The current indicators are placed outside of the bore so that at times the patient is not able to see them easily, regardless of whether a feet first scan or a head first scan is being performed. The indicators are stationary, so when a patient table starts moving during surview and clinical scans the indicator will no longer be visible even if they were initially visible. For a myopic patient, it may also be difficult to see the indicator as the patient will not be wearing glasses during the scan.

Thus, there is a need for an improved way to provide breath hold instructions to a patient during a scan.

### SUMMARY OF THE INVENTION

The invention is defined by the claims.

According to examples in accordance with an aspect of the invention, there is provided a medical scanner, comprising:
a scanning system comprising a scanner housing having a bore through which a patient is moved during a scan; and
an instruction system for providing breathing instructions to a patient during a scan,
wherein the instruction system comprises a colored lighting strip which extends along part of a front face of the scanner housing, along an internal surface of the bore (16) and along part of a rear face of the scanner housing.

In this way, intuitive instructions can be provided as color outputs, and they can be seen by the patient while the patient is moved through the bore. The color outputs can be seen for both feet first and head first scans, and they do not require the patient to have good vision. The lighting strip is visible from either side of the scanner housing as well as from within the scanner bore.

The lighting strip for example comprises a LED strip. This is a low-cost lighting solution which can easily be controlled to output different colors as well as temporal and/or spatial lighting patterns if desired. The lighting strip for example comprises an array of LEDs and a diffuser over the array of LEDs. The lighting strip is for example a flexible LED strip so that it can be applied to the scanner housing and adopt the external shape of the scanner housing. It may thus be provided as a retrofit solution.

The lighting strip for example extends along a highest portion of the internal surface of the bore. Thus, it can be seen by the patient when looking directly upwards when their head is within the bore.

The medical scanner for example comprises a controller for controlling the lighting strip, wherein the controller is configured to control the lighting strip to provide a first output type with a first color to provide an instruction for anticipating a breath hold, a second output type with a second color to provide an instruction for a breath hold and a third output type with a third color to provide an instruction for normal breathing after a breath hold.

Thus, different colors represent different breathing instructions.

The first color is for example yellow, the second color is red, and the third color is green. Thus, the lighting strip provides an intuitive traffic light output (green is breathe, yellow is prepare for breath hold and red is breath hold i.e., do not breathe).

The third output type (for normal breathing) is for example a pulsating output. It may in this way also provide breathing rate guidance to encourage relaxed breathing.

The instruction system preferably further comprises a speaker, wherein the controller is for controlling the speaker and is configured to control the speaker to provide audio during the first to third outputs. The audio outputs supplement the color outputs.

The audio output during the second output type (the breath hold phase) for example comprises a countdown of the required duration of the breath hold.

The audio output during the third output type (the normal breathing phase) for example comprises a soundscape to represent inhalation and exhalation phases. This provides a further stimulus to provide breathing rate guidance to encourage relaxed breathing.

The controller is for example configured to control the lighting strip to provide the third output type (to represent normal breathing) before the output which indicates anticipation of a breath hold. Thus, the normal breathing instruction is used before and after each breath hold. There may for example be multiple breath hold times during the overall scan procedure.

Thus, there may be considered to be a four-cycle sequence using the three types of output, of normal breathing (in preparation for a breath hold), anticipation of a breath bold, breath hold and normal breathing again (after the breath-bold).

A fourth output type may be used to indicate the end of the scan procedure, for example a black output.

In some examples, the scanning system is a CT scanning system. In other examples, the scanning system is a MRI scanning system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a known medical scanner; and
Fig. 2 shows a medical scanner with an instruction system of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a medical scanner comprising a scanning system with a scanner housing having a bore through which a patient is moved during a scan. An instruction system is used for providing breathing instructions to the patient during a scan. The instruction system comprises a colored lighting strip which extends from outside the bore at opposite sides of the housing through the bore. In this way, intuitive instructions can be provided as color outputs, and they can be seen by the patient while the patient is moved through the bore.

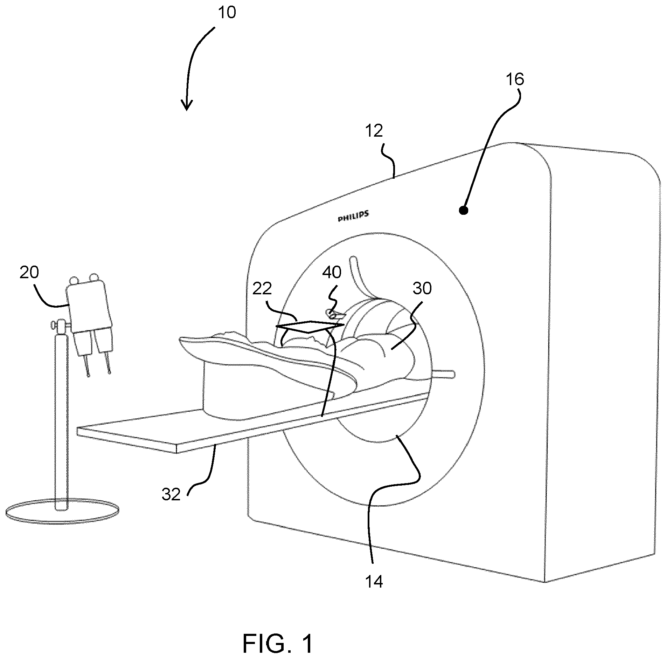

Fig. 1 shows a known medical scanner 10 comprising a scanning system 12. The scanning system comprises a housing through which a bore 14 extends. The scanning system is for example a CT scanner. The housing has a front face 16 and a rear face (not seen in Fig. 1).

The medical scanner also has a contrast injection system 20 for injecting a contrast agent for contrast scans.

A system is provided for presenting instructions or advice to the patient during the scan, such as a color display panel 22 mounted inside the bore and associated speaker.

The scanner has a couch 32 that moves through the bore 14, and a patient 30 is shown on the couch. A monitoring system 40 is also shown for monitoring various vital signs and other patient information. The monitoring system 40 for example comprises at least a camera. The monitoring system for example monitors one or more of heart rate (using remote PPG implemented using a camera), blood pressure (using remote PPG implemented using a camera), muscle tension (using galvanic skin response electrodes), and facial expression (using camera image analysis).

A typical CT exam involves a surview, which is a low dose trial exposure to identify the area of interest for the clinical scan, and one or many clinical scans with or without the use of a contrast agent.

For a breath-hold scan the patient is supposed to perform an inhalation and then hold their breath upon receiving a breath hold instruction (e.g., via the display panel 22 or by audio instruction). The breath hold is critical for the clinical scans, whereas the surview is used to educate the patient in practicing the breath-hold for the subsequent clinical scan, which is to be used for interpretation purposes.

The display 22 is used to guide the patient throughout the exam to enable anticipation of breath hold and to instruct the start and stop (and duration) of the breath hold.

This invention relates to an alternative way to provide the breath hold instructions to the user. It may be used instead of the display 22, but equally it may be used in combination with other indicator systems such as the display 22.

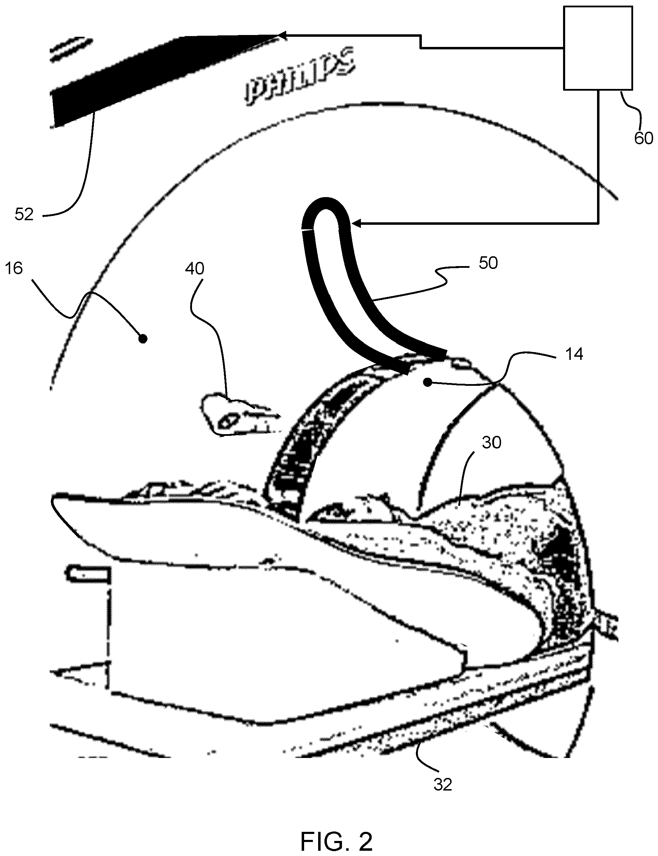

Fig. 2 shows an enlarged area of the scanner housing and bore and shows an instruction system in accordance with the invention for providing breathing instructions to a patient during a scan. The instruction system comprises a colored lighting strip 50 which extends from outside the bore (at both ends of the bore) to within the bore. It extends through the bore in the patient couch movement direction (i.e., a longitudinal direction).

The lighting strip extends along part of the front face 16 of the scanner housing and into the inner surface of the bore. In particular, it extends downwardly from a first end of the strip at the front face 16 from above the bore, and into the bore. It then extends along an internal surface of the bore, in particular along the highest section of the roof of the bore. It also extends along part of a rear face of the scanner housing, in particular upwardly to the opposite second end of the strip above the bore. Thus, the lighting strip is generally symmetric between the front and rear faces. The lighting strip extends all the way through the bore and upwardly from the bore at the two ends. In this way, it is visible in the same way for head-first and feet-first patients. The lighting strip is visible from either side of the scanner housing as well as from within the scanner bore.

The lighting strip comprises a LED strip, comprising a diffusive cover over a LED array. The lighting strip is for example a flexible LED strip so that it can be retro-fitted to existing scanner housings and adopt the external shape of the scanner housing and bore. The lighting strip may comprise a solid area of lighting, or it may comprise a peripheral band of lighting.

In the example shown, there is additionally an optional second arcuate lighting strip 52 which extends concentrically around the bore, at the front and back surfaces (only the front surface is shown). The arcuate strip for example extends around only the upper portion of the bore where it will be visible to a patient, thus forming a semi-circular arc.

The lighting strip (or strips) is (or are) controlled by a controller (shown schematically as 60) to provide different color outputs to provide different breathing instructions.

For example, a first output type with a first color is used to provide an alert to the user of an imminent breath hold, thus enabling the user to anticipate the breath hold. This may be a yellow (or amber) color, to represent an amber traffic light (prepare to stop breathing). A second output type with a second color is used to provide an instruction for a breath hold. This may be a red color, to represent a red traffic light (stop breathing). A third output type with a third color is to provide an instruction for breathing after a breath hold. This may be a green color to represent a green traffic light (breathe). The third output type is also used before the anticipation of a breath hold. Thus, the patient is guided to breath normally in a relaxed manner before and after a breath hold. Thus, there may be considered to be four cycles - normal breathing, anticipation, breath-hold and normal breathing,

The third, green, output type may pulsate to provide breathing rate guidance to encourage relaxed breathing.

The instruction system also has a speaker, and the controller drives the speaker to provide audio output during the outputs using the first to third output types. For example, during the breath hold phase, a countdown may be provided of the required duration of the breath hold. During the breathe normally phase, the audio output may provide a soundscape to represent inhalation and exhalation phases. This provides a further stimulus to provide breathing rate guidance to encourage relaxed breathing.

There may be multiple breath hold times during the overall scan procedure. The end of the scan procedure may be notified to the patient by a fourth output, for example a black color. The LED strip is turned off when the system is idle.

The sequence of operation will now be explained based on the example above. As soon as patient is moved inside the bore, the LED strip 50 turns on with the pulsating green output mimicking respiration. An audible soundscape of inhale and exhale is provided in sync with the pulsating green color to guide the patient into calm breathing. This continues until the operator selects the exam protocol, starts the exam and presses "Go" to initialize the gantry.

When operator presses "X-Ray release", the LED strips turns yellow indicating the anticipation to breath-hold, and voice instructions are also given to the patient to "Breathe In & Hold your breath" (in a chosen language).

After 4 seconds (for example) of delay of the X-Ray release and finish of the voice command, the LED strip turns solid red to indicate breath hold.

The scanner then starts the exposure, and the patient table moves automatically collecting slices. During this movement, the LED strip remains visible and the patient feels more aware about the status of scan. An audible countdown helps the patient to know the estimated time remaining in the scan.

Once the active scan is finished, the LED strip turns back to the pulsating green, along with the inhale and exhale assistance audio. A voice command may also be used to advise the patient to breathe again. The operator then does the planning for the next scan.

Once the last scan is finished, the lighting strip provides the pulsating green output for a period of time (e.g., 4 seconds) and a voice command advises the patient to breathe again. The lighting strip then turns black indicating the end of the exam and the patient table is moved out and lowered automatically.

The invention has been described with reference to a CT scan. However, the invention may also be applied to other imaging types involving movement of a patient through a scanner bore such as MRI scans, digital X-ray radiogrammetry as well as radiotherapy and image guided therapy.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A medical scanner, comprising:
a scanning system comprising a scanner housing having a bore (16) through which a patient is moved during a scan; and
an instruction system (50,52) for providing breathing instructions to a patient during a scan,
wherein the instruction system comprises a colored lighting strip (50) which extends along part of a front face (14) of the scanner housing, along an internal surface of the bore (16) and along part of a rear face of the scanner housing.

2. The medical scanner of claim 1, wherein the lighting strip comprises a LED strip.

3. The medical scanner of claim 2, wherein the lighting strip comprises an array of LEDs and a diffuser over the array of LEDs.

4. The medical scanner of any one of claims 1 to 3, wherein the lighting strip extends along a highest portion of the internal surface of the bore.

5. The medical scanner of any one of claims 1 to 4, comprising a controller (60) for controlling the lighting strip, wherein the controller is configured to control the lighting strip to provide a first output type with a first color to provide an instruction for preparation for a breath hold, a second output type with a second color to provide an instruction for a breath hold and a third output type with a third color to provide an instruction for breathing after a breath hold.

6. The medical scanner of claim 5, wherein the first color is yellow, the second color is red, and the third color is green.

7. The medical scanner of any one of claims 1 to 6, wherein the third output type is a pulsating output.

8. The medical scanner of any one of claims 5 to 7, wherein the instruction system further comprises a speaker, wherein the controller is for controlling the speaker and is configured to control the speaker to provide audio output during the output using the first to third output types.

9. The medical scanner of claim 8, wherein the audio output during the second output type comprises a countdown of the required duration of the breath hold.

10. The medical scanner of claim 7 or 8, wherein the audio output during the third output type comprises a soundscape to represent inhalation and exhalation phases.

11. The medical scanner of any one of claims 5 to 10, wherein the controller is configured to control the lighting strip to provide the third output type to represent normal breathing before preparation for a breath hold.

12. The medical scanner of any one of claims 1 to 11, wherein the scanning system is a CT scanning system.

13. The medical scanner of any one of claims 1 to 11, wherein the scanning system is a MRI scanning system.
